# EUROPÄISCHE PATENTSCHRIFT

(11) **EP 1 926 480 B1**
(45) Veröffentlichungstag und Bekanntmachung des Hinweises auf die Patenterteilung: **17.07.2013**
(21) Anmeldenummer: 06793103.0
(22) Anmeldetag: 31.08.2006
(51) Int. Cl.: A61K 9/48

(54) **Verfahren zur Herstellung von Weichkapselhüllen auf Basis von Polyvinylalkohol-Polyethylenglykol-Pfropfcopolymeren**
Method for the production of soft capsule envelopes based on polyvinyl alcohol-polyethylene glycol graft copolymers
Procédé pour produire des enveloppes de capsules molles a base de copolymères greffés alcool polyvinylique / polyéthylèneglycol

(30) Priorität: 09.09.2005 DE 102005043172
(43) Veröffentlichungstag der Anmeldung: 04.06.2008
(73) Patentinhaber: BASF SE, 67056 Ludwigshafen (DE)
(72) Erfinder: KOLTER, Karl, 67117 Limburgerhof (DE); MITTWOLLEN, Jan-Peter, 68535 Edingen-Neckarhausen (DE); SCHILLO, Simone, 67069 Ludwigshafen (DE); MEYER-BÖHM, Kathrin, 90537 Feucht (DE); ANGEL, Maximilian, 67105 Schifferstadt (DE)
(86) Internationale Anmeldenummer: PCT/EP2006/065865
(87) Internationale Veröffentlichungsnummer: WO 2007/028758

(56) Entgegenhaltungen:
- EP-A1- 1 136 070
- WO-A-99/40156
- DE-A1- 2 363 853
- DE-B- 1 081 229
- DE-B- 1 094 457
- GB-A- 2 361 643

## Beschreibung

Die vorliegende Erfindung betrifft ein verbessertes Verfahren zur Herstellung von Weichkapselhüllen auf Basis von Polyvinylester-Polyethylenglykol-Pfropfcopolymeren.

Weichkapseln zeichnen sich dadurch aus, dass die Herstellung der Hülle und das Befüllen in einem Schritt nahezu simultan erfolgen. In der Regel besteht die Hülle solcher Weichkapseln hauptsächlich aus Gelatine, weshalb die Kapseln auch häufig als Weichgelatinekapseln bezeichnet werden. Da Gelatine an sich ein sprödes, wenig flexibles Material ist, muss es entsprechend weichgemacht werden, das heißt es müssen Weichmacher zugesetzt werden. Solche Weichmacher sind niedermolekulare Verbindungen, in der Regel Flüssigkeiten, wie z.B. Glycerin, Propylenglykol, Polyethylenglykol 400. Darüber hinaus enthalten solche Kapseln oft noch Farbstoffe, Opakisierungsmittel und Konservierungsstoffe.

Gelatine wird zwar häufig eingesetzt, jedoch weist sie zahlreiche Nachteile auf. So ist Gelatine ein Material tierischen Ursprungs und damit nicht kosher. Außerdem bleibt immer ein geringes Restrisiko von BSE, da zu ihrer Herstellung bevorzugt Gelatine von Rindern verwendet wird. Die Gewinnung einer geeigneten Gelatine ist sehr aufwendig und erfordert eine strenge Überwachung des Prozesses. Trotzdem sind die Chargenunterschiede aufgrund des tierischen Ursprungs, der einer gewissen Variabilität unterliegt, groß. Gelatine ist mikrobiell sehr anfällig, da sie einen guten Nährboden für Mikroorganismen darstellt. Bei der Herstellung, wie auch der Verwendung von solchen Verpackungsmaterialien müssen deshalb entsprechende Maßnahmen ergriffen werden. Häufig ist der Einsatz von Konservierungsmitteln unerlässlich.

Die bei der Herstellung von Gelatinekapseln unbedingt erforderlichen Weichmacher treten häufig von der Hülle in das Füllgut über und führen dort zu Veränderungen. Die Hülle verarmt an Weichmachern und wird im Laufe der Lagerung spröde und mechanisch instabil. Darüber hinaus besitzt eine Weichgelatinekapsel einen relativ hohen Wassergehalt in der Hülle, der ebenfalls eine weichmachende Wirkung hat. Werden solche Kapseln bei reiner Luftfeuchte gelagert, so verdunstet Wasser aus der Hülle, wodurch die Kapsel ebenfalls versprödet. Gleiches passiert auch, wenn sehr hygroskopische Güter verkapselt werden. Besonders hygroskopische oder hydrolyseempfindliche Stoffe können überhaupt nicht verkapselt werden.

Die Lösungsgeschwindigkeit von Gelatine ist verhältnismäßig langsam. Für schnelle Wirkstofffreisetzungen wäre eine höhere Auflösungsgeschwindigkeit in Magen- bzw. Darmsaft wünschenswert.

Zahlreiche Stoffe führen mit Gelatine zu Interaktionen wie z.B. Aldehyde, Polyphenole, reduzierende Zucker, mehrwertige Kationen, Elektrolyte, kationische oder anionische Polymere etc., wobei häufig Vernetzung eintritt und die Kapsel nicht mehr oder nur noch ganz langsam zerfällt bzw. sich auflöst. Für ein Arzneimittel sind solche Veränderungen verheerend, da die Wirksamkeit nicht mehr gegeben ist. Auch viele Arzneistoffe führen mit Gelatine zu Interaktionen. Zum Teil bilden sich während der Lagerung Abbauprodukte von Arzneistoffen mit beispielsweise aldehydischer Struktur, die zu einer Vernetzung der Gelatine führen. Da Gelatine sowohl saure wie auch basische Gruppen aufweist, ist verständlich, dass Reaktionen mit anderen geladenen Molekülen leicht eintreten.

Gelatine kann enzymatisch gespalten werden. Verunreinigungen durch Enzyme bzw. von Bakterien abgesonderte Enzyme können die Eigenschaften von Gelatine dramatisch verändern.

Weichgelatinekapseln verkleben sehr leicht unter warmen und feuchten Bedingungen.

Die Haftung von Filmüberzügen auf Weichgelatinekapseln ist extrem schlecht. Häufig muss hierbei umständlich erst ein spezielles Subcoating aufgezogen werden.

Aufgrund dieser vielen Nachteile hat es nicht an Versuchen gefehlt, die Gelatine in Weichkapseln ganz oder teilweise zu ersetzen.

So werden beispielsweise in der US 6,340,473 Weichkapseln auf Basis von modifizierten Stärken und Carrageenanen beschrieben.

Polyvinylalkohol ist beispielsweise für diesen Zweck beschrieben. Polyvinylalkohol weist jedoch eine langsame Lösungsgeschwindigkeit auf, erfordert ebenfalls zusätzliche Weichmacher, die wiederum migrieren können und die, wie oben bereits beschrieben, die Eigenschaften des Füllguts verändern können, und kann außerdem in Folge innerer Kristallisation stark verspröden. Insbesondere bei niedriger Umgebungsfeuchte nimmt die Flexibilität im Laufe der Lagerung dramatisch ab.

DE-A2 2 363 853 beschreibt die Verwendung von teilverseiften Copolymerisaten von Vinylacetat auf Polyethylenglykol zur Herstellung von Hartkapseln für Medikamente. Für die Verwendung der Copolymerisate zur Herstellung von Weichkapseln finden sich in dieser Schrift keine Hinweise.

An Hartkapseln werden jedoch ganz andere Anforderungen gestellt als an Weichkapseln. Hartkapseln benötigen eine hohe Festigkeit während bei Weichkapseln die Flexibilität im Vordergrund steht. Auch die Herstellungsverfahren sind völlig unterschiedlich. Bei Hartkapseln wird zunächst nur die Hülle in 2 separaten Teilen, einem Oberteil und Unterteil, mittels eines Tauchverfahrens hergestellt, während bei Weichkapseln die Herstellung der Hülle und die Füllung nahezu simultan verlaufen.

Bei den Hartkapseln werden nach der Herstellung von Oberteil und Unterteil diese locker ineinandergeschoben, so dass der pharmazeutische Hersteller die beiden Teile maschinell wieder trennen kann, sein Pulver einfüllen und die Kapsel verschließen kann. Bei näherer Betrachtung dieser Verarbeitung ist klar, dass die beiden Kapselteile mechanisch sehr stabil sein müssen, zumal die Füllmaschinen sehr schnell laufen und Formveränderungen den ganzen Prozess lahm legen würden.

Bei Weichkapseln muss die Hülle erstens absolut dicht sein, damit das Füllgut, das in der Regel flüssig ist, nicht austreten kann, und zweitens sehr flexibel sein, weil das Füllgut sonst durch Risse bzw. Mikrorisse austreten würde. Bei der Herstellung ist eine besonders hohe Flexibilität erforderlich, weil der Polymerfilm in Hohlbohrungen eingesaugt wird und damit stark verformt und gedehnt wird. Die Herstellung von Weichkapseln ist ein technologisch enorm anspruchsvoller Prozess, von daher müssen die Polymereigenschaften und die Maschinen exakt angepasst und eingestellt werden.

Die völlig unterschiedlichen Verfahren zur Herstellung von Hart- und Weichgelatinekapseln sind beschrieben in: W. Fahrig und U. Hofer, Die Kapsel, Wissenschaftliche Verlagsgesellschaft mbH Stuttgart, 1983, S. 58-82.

Die Anmeldung WO 97/35537 beschreibt ein spezielles Verfahren zur Herstellung von Weichkapseln unter Verwendung von verschiedenen Materialien, hauptsächlich Polyvinylalkohol. Vor der Verkapselung wird ein Lösungsmittel auf den Film aufgebracht, um ihn anzulösen, damit die Verklebung besser erfolgen kann. Dies ist allerdings nur bei entsprechend schwer zu verarbeitenden Filmen erforderlich.

DE 1 094 457 und DE 1 081 229 beschreiben Verfahren zur Herstellung von Pfropfpolymerisaten von Polyvinylalkohol auf Polyalkylenglykolen durch Verseifung der Vinylester und deren Verwendung als Schutzkolloide, wasserlösliche Verpackungsfolien, als Schlichte- und Appreturmittel für Textilien und in der Kosmetik.

In WO 99/40156 werden Kombinationen von Polyethylenglykolen unterschiedlicher Molekulargewichte beschrieben, die für die Herstellung von Filmen bzw. Weichkapseln geeignet sind. Polyethylenglykole mit hohem Molekulargewicht lösen sich aber nur langsam in Wasser auf und sind spröde. Durch die Kombination mit Polyethylenglykolen mit sehr niedrigem Molekulargewicht werden sie zwar etwas flexibler aber auch klebriger. Zudem können diese wiederum aufgrund ihres niedrigen Molekulargewichtes in das Füllgut migrieren.

Gemäß der US-A 3,984,494 werden Polyvinylalkohol-Polyether-Pfropfpolymere für die Herstellung von Hartkapseln beschrieben.

In der EP-A 1136070 sind Weichkapseln aus Polyvinylalkohol-Polyether-Pfropfpolymeren beschrieben. Zur Herstellung der Kapselhüllen werden die Polymere zunächst in Wasser gelöst und dann zu Filmen ausgezogen. Allerdings lassen die so erhaltenen Filme hinsichtlich ihrer Festigkeit und Verarbeitungsfähigkeit noch Raum für Verbesserungen.

Aufgabe der vorliegenden Erfindung war es, ein verbessertes Verfahren zur Herstellung von Weichkapselhüllen zu finden.

Demgemäss wurde ein Verfahren zur Herstellung von Weichkapselhüllen auf Basis von Polyvinylakohol-Polyether-Pfropfpolymeren gefunden, welches dadurch gekennzeichnet ist, dass eine wässrige Lösung, enthaltend mindestens 45 Gew.-% einer Polymerkomponente, welche aus Polyvinylalkohol-Polyether- Pfropfpolymer oder einer Mischung des Polyvinylalkohol-Polyether- Pfropfpolymers mit Polyvinylalkohol besteht, mit einer Temperatur von mindestens 70°C extrudiert und die Extrudate durch Abkühlung zu Filmen verfestigt werden.

Bevorzugte erfindungsgemäß verarbeitete Pfropf-Polymere sind erhältlich durch radikalische Polymerisation von

| | |
|---|---|
| a) 10 bis 98 Gew.-% | mindestens eines Vinylesters von C₁-C₂₄-Carbonsäuren in Gegenwart von |
| | |
| b) 2 bis 90 Gew.-% | mindestens einer polyetherhaltigen Verbindung und |

anschließender Verseifung der Estergruppen.

Besonders bevorzugt verarbeitet werden Polymere, die durch radikalische Polymerisation von

| | |
|---|---|
| a) 50 bis 97 Gew.-% | mindestens eines Vinylesters von C₁-C₂₄-Carbonsäuren in Gegenwart von |
| | |
| b) 3 bis 50 Gew.-% | mindestens einer polyetherhaltigen Verbindung |

erhältlich sind.

Ganz besonders bevorzugt verarbeitet werden Polymere, die erhältlich sind durch radikalische Polymerisation von

| | |
|---|---|
| a) 65 bis 97 Gew.% | mindestens eines Vinylesters von C₁-C₂₄-Carbonsäuren in Gegenwart von |
| | |
| b) 3 bis 35 Gew.% | mindestens einer polyetherhaltigen Verbindung und |

als Vinylester wird bevorzugt Vinylacetat eingesetzt.

Das Molekulargewicht der Polyether liegt im Bereich kleiner 500000 (nach Zahlenmittel), bevorzugt im Bereich von 300 bis 100000, besonders bevorzugt im Bereich von 500 bis 50000, ganz besonders bevorzugt im Bereich von 1000 bis 20000.

Vorteilhafterweise verwendet man Homopolymerisate des Ethylenoxids oder Copolymerisate, mit einem Ethylenoxidanteil von 40 bis 99 Gew.-%. Für die bevorzugt einzusetzenden Ethylenoxidpolymerisate beträgt somit der Anteil an einpolymerisiertem Ethylenoxid 40 bis 100 mol-%. Als Comonomer für diese Copolymerisate kommen Propylenoxid, Butylenoxid und/oder Isobutylenoxid in Betracht. Geeignet sind beispielsweise Copolymerisate aus Ethylenoxid und Propylenoxid, Copolymerisate aus Ethylenoxid und Butylenoxid sowie Copolymerisate aus Ethylenoxid, Propylenoxid und mindestens einem Butylenoxid. Der Ethylenoxidanteil der Copolymerisate beträgt vorzugsweise 40 bis 99 mol-%, der Propylenoxidanteil 1 bis 60 mol-% und der Anteil an Butylenoxid in den Copolymerisaten 1 bis 30 mol-%. Neben geradkettigen können auch verzweigte Homo- oder Copolymerisate als polyetherhaltige Verbindungen b) verwendet werden.

Zur Herstellung der erfindungsgemäß verwendeten Pfropf-Polymeren werden die Estergruppen der ursprünglichen Monomere a) und gegebenenfalls weiterer Monomere nach der Polymerisation durch Hydrolyse, Alkoholyse oder Aminolyse gespalten. Im Nachfolgenden wird dieser Verfahrensschritt allgemein als Verseifung bezeichnet. Die Verseifung erfolgt in an sich bekannter Weise durch Zugabe einer Base, bevorzugt durch Zugabe einer Natrium- oder Kaliumhydroxidlösung in Wasser und/oder Alkohol. Besonders bevorzugt werden methanolische Natrium- oder Kaliumhydroxidlösungen sowie Natrium- oder Kaliummethanolatlösungen eingesetzt, Die Verseifung wird bei Temperaturen im Bereich von 10 bis 80°C, bevorzugt im Bereich von 20 bis 60°C, durchgeführt. Der Verseifungsgrad hängt ab von der Menge der eingesetzten Base, von der Verseifungstemperatur, der Verseifungszeit und dem Wassergehalt der Lösung.

Der Verseifungsgrad der Polyvinylestergruppen liegt im Bereich von 50 bis 100 %, bevorzugt im Bereich von 60 bis 100 % und besonders bevorzugt im Bereich von 80 bis 100 %.

Insbesondere lässt sich mit Hilfe des erfindungsgemäßen Verfahrens ein Pfropfpolymer verarbeiten, das durch Polymerisation von 15 Gew.-% Polyetheranteil und 85 Gew.-% Vinylacetat erhalten wurde. Das Molekulargewicht der Polyetherkomponente beträgt dabei bevorzugt 6000 Dalton. Der Verseifungsgrad beträgt bevorzugt 88-98 %.

Als weitere Polymer-Komponente können die Weichkapselhüllen Polyvinylalkohol enthalten. Bevorzugt werden Polyvinylalkohole mit mittleren Molekulargewichten M_{w} von 1000 bis 500000, bevorzugt 5000 bis 100000 und besonders bevorzugt 10000 bis 50000 eingesetzt. Das Gewichtsverhältnis von Polyvinylester-Polyether-Pfropfpolymeren zu Polyvinylalkoholen kann 100:0 bis 30:70, bevorzugt 100:0 bis 50:50, betragen.

Zur Durchführung des erfindungsgemäßen Verfahrens wird zunächst eine Lösung der Pfropfpolymeren oder der Mischung des Pfropfpolymers mit dem Polyvinylakohol und gegebenenfalls weiterer Hilfsstoffe hergestellt, wobei der Feststoffgehalt der Lösung so gewählt wird, dass der Gehalt an Pfropfpolymeren oder an der Summe aus Pfropfpolymeren und Polyvinylalkohol mindestens 45 Gew.-% und bis zu 75 Gew.-%, bevorzugt 50 bis 70 Gew.-%, bezogen auf das Gesamtgewicht der wässrigen Lösung, beträgt. Die Lösung wird vor der Extrusion auf eine Temperatur von mindestens 70°C und bis zu 120°C, bevorzugt 75 bis 100°C gebracht. Anschliessend wird die heiße Lösung ausgetragen und nach Abkühlung ein Film erzeugt.

Als geeignete Vorrichtungen zur Durchführung des Verfahrens kommen übliche Extruder in Betracht. Weiterhin eignet sich jede andere Vorrichtung, die zur Verarbeitung von heißen Flüssigkeiten ausgelegt ist und über eine Pumpe oder andere Fördereinheiten verfügt, um die heiße polymerhaltige Flüssigkeit unter Druck durch eine geeignete Austragsvorrichtung zu pressen. Es können hierbei alle üblichen Pumpen und Fördereinheiten verwendet werden, die in der Lage sind, pulsationsfrei zu fördern.

Bevorzugt lässt sich das erfindungsgemäße Verfahren mit Hilfe eines Extruders durchführen. Für das erfindungsgemäße Verfahren eignen sich prinzipiell die üblichen, dem Fachmann bekannten Extrudertypen. Diese umfassen üblicherweise ein Gehäuse, eine Antriebseinheit sowie eine Plastifizier- oder Mischeinheit aus einer oder mehreren mit Förder- oder Mischelementen versehenen rotierenden Achsen (Schnecken).

Längs der Schnecken erstrecken sich in Transportrichtung mehrere Abschnitte, die im erfindungsgemäßen Verfahren eine Einzugszone, eine Mischzone und eine Ausstoßzone umfassen. Weiterhin können gewünschtenfalls auch Entgasungszonen vorgesehen sein, wobei die Entgasung bei Atmosphärendruck und/oder Vakuum erfolgen kann. Die Vakuumentgasung kann beispielsweise mit Hilfe einer Stopfschnecke und einer Dampfstrahlpumpe erfolgen.

Jeder dieser Abschnitte kann wiederum einen oder mehrere Zylinder (Schüsse) als kleinste unabhängige Einheit enthalten.

Die Herstellung der zu extrudierenden Lösung kann in einem Einschneckenextruder , einem Zweischneckenextruder oder in Mehrschneckenextrudern erfolgen, bevorzugt aber in einem Zweischneckenextruder. Mehrere Schnecken können gleichsinnig oder gegensinnig drehend, kämmend oder dicht kämmend ausgeführt sein. Der Extruder ist vorzugsweise gleichsinnig dicht kämmend ausgelegt. Die einzelnen Zylinder sollen beheizbar sein.

Die Schnecken können aus allen in der Extrusion üblichen Elementen aufgebaut sein. Sie können neben üblichen Förderelementen auch Knetscheiben oder Rückförderelemente enthalten, um eine optimale Durchmischung der Komponenten zu gewährleisten. Welche Schneckenkonfiguration im Einzelfall geeignet ist, kann der Fachmann durch einfache Versuche ermitteln. Das Verhältnis von Schraubenlänge zu Schraubendurchmesser (LD-Verhältnis) kann 20 :1 bis 40 :1 betragen vorzugsweise 24 :1 bis 36 :1.

Der erfindungsgemäß verwendete Extruder gliedert sich im wesentlichen in folgende Abschnitte auf:
Eine erste Zone dient als Rückwärtsentgasungszone, um eine Entlüftung der durch die pulverförmige Polymerkomponente eingeschleppten Luft zu ermöglichen. In einer zweiten Zone wird die Polymerkomponente mittels geeigneten Dosiervorrichtungen wie beispielsweise Differentialdosierwaagen in den Extruder dosiert. An diese Zone kann sich eine Förderzone anschließen, in der die Polymerkomponente gewünschtenfalls angewärmt werden kann. Daran schließt sich eine Zone an, in der Wasser und gegebenenfalls weitere Flüssigkomponenten über Kolben- oder Zahnradpumpen zudosiert werden. In den sich daran anschließenden Zonen erfolgt eine intensive Vermischung der Komponenten. Diese Zonen sind vorzugsweise mit Knetscheiben ausgerüstet, um eine ausreichende Durchmischung zu gewährleisten. Alle Zonen sind beheizbar. Üblicherweise wird eine. Manteltemperatur von 90 bis 120°C gewählt. Der sich einstellende Extrusionsdruck liegt im Bereich von 0,05 bis 0,2 MPa.

Der Austrag der heißen Polymerlösung erfolgt wie beschrieben durch eine geignete Austragsöffnung. Der Austrag kann beispielsweise über eine Rohrdüse oder vorzugsweise über eine Schlitzdüse erfolgen. Bei Verwendung von Rohrdüsen kann der austretende Extrudatstrang anschliessend durch Ausrakeln oder mit Hilfe von Walzenstühlen verformt werden.

Schlitzdüsen zur Herstellung von Filmen sind an sich bekannt. Geeignete Schlitzhöhen weisen einen Schlitzdurchmesser von 100 bis 2000 µm, vorzugsweise von 150 bis 1000 µm, auf.

Nach Verlassen der Schlitzdüse werden die Filme durch ein laufendes Förderband oder Walzen weitertransportiert. Hierbei werden die Extrudate durch Abkühlen verfestigt. Dabei wird die Temperatur des Extrudats um mindestens 10°C abgesenkt, um eine Verfestigung zu Filmen zu erzielen. Die Abkühlung der Filme kann durch eine entsprechende Kühlung des Förderbandes erfolgen oder auch durch kalte Luft, die über den Film geleitet wird. Es können auch beide Techniken miteinander kombiniert werden. Geeignete Filmdicken liegen zwischen 100 und 2000 µm, vorzugsweise zwischen 150 und 1000 µm.

Die erfindungsgemäß hergestellten als Weichkapselhüllen geeigneten Filme weisen insbesondere folgende Eigenschaften auf:
- Reißdehnung:: 50 - 600 % in feuchtem Zustand
25 - 300 % in getrocknetem Zustand (bei einer Gleichgewichtsfeuchte von 53 % relativer Feuchte.)
- Zugfestigkeit:: 2 - 30N/mm2 in feuchtem Zustand
5 - 60N/mm2 in getrocknetem Zustand (bei einer Gleichgewichtsfeuchte von 53 % relativer Feuchte)

Neben den Polyvinylalkohol-Polyether-Pfropfpolymeren und gegebenenfalls dem Polyvinylalkohol können den zur Herstellung der Weichkapselhüllen verwendeten Lösungen noch weitere Hilfsstoffe zugegeben werden.

So können den Lösungen 0,1 bis 30 Gew.-%, bezogen auf das Gesamtgewicht der Lösungen, an Weichmachern, beispielsweise Glycerin, 1,2 Propylenglykol oder Polyethylenglykol mit Molekulargewichten zwischen 250 und 600 zugegeben werden.

Zur Erzielung einer Magensaftresistenz können in der Hülle 20 bis 80 %, vorzugsweise 30 bis 70 % eines magensaftresistenten Polymers enthalten sein.

Den Polymerisaten können strukturverbessernde Hilfsstoffe zugesetzt werden, um die mechanischen Eigenschaften wie Flexibilität und Festigkeit zu modifizieren. Diese strukturverbessernden Hilfsstoffe lassen sich in 2 große Gruppen einteilen.
A) Polymere mit einem Molekulargewicht größer 50000, vorzugsweise größer 100000
B) Stoffe die zu einer Vernetzung der Polymerketten entweder der Polymeren oder der unter A) genannten Stoffe führen, vorzugsweise Aldehyde, Borsäure und ihre Salze,
sowie gegebenenfalls Stoffe, die zu einer Vernetzung der Polymerketten der strukturverbessernden Hilfsstoffen führen, vorzugsweise Erdalkaliionen, Amine, Tannine sowie Aldehyde und Borate.

Als Polymere mit hohem Molekulargewicht können Stoffe aus folgenden Stoffklassen eingesetzt werden:
Polyaminosäuren, wie Gelatine, Zein, Sojaprotein sowie Derivate davon,
Polysaccharide wie Stärke, abgebaute Stärke, Maltodextrine, Carboxymethylstärke, Hydroxypropylstärke, Cellulose, Hydroxypropylmethylcellulose, Hydroxypropylcellulose, Hydroxyethylcellulose, Methylcellulose, Carboxymethylcellulose, Ethylcellulose, Celluloseacetat, Celluloseacetatphthalat, Hydroxypropylcelluloseacetatphthalat, Hydroxypropylcelluloseacetatsuccinat, Hemicellulose, Galactomannane, Pectine, Alginate, Carrageenane, Xanthan, Gellan, Dextran, Curdlan, Pullulan, Chitin, sowie Derivate davon, synthetische Polymere wie Polyacrylsäure, Polymethacrylsäure, Copolymerisate aus Acrylsäure- und Methacrylsäureestern, Polyvinylacetat, Polyethylenglykole, Polyoxyethylen-Polyoxypropylen-Blockpolymere, Polyvinylpyrrolidone sowie Derivate davon.

Diese Polymere mit hohem Molekulargewicht bilden ein Netzwerk mit den Polymeren und erhöhen so die Festigkeit der Weichkapseln. Die Flexibilität leidet, sofern keine sehr hohen Konzentrationen verwendet werden in aller Regel nicht. Überraschenderweise sind hierfür nicht nur wasserlösliche, sondern auch wasserunlösliche Polymere wie Copolymere aus Acrylsäure- und Methacrylsäureestern geeignet. Bleibt die Konzentration dieser wasserunlöslichen Polymere unter 50 %, zerfallen die Kapseln immer noch.

Bevorzugte strukturverbessernde Hilfsstoffe sind Carrageenane, modifizierte Stärken wie Hydroxypropylstärke oder Cellulosederivate wir Hydroxypropylcellulose.

In ähnlicher Weise wirken Stoffe, die zu einer Vernetzung entweder der Polymerketten der Polymere oder der zugesetzten hochmolekularen Polymere führen.

Neben den genannten Komponenten können die erfindungsgemäßen Weichkapselhüllen noch weitere übliche Bestandteile enthalten. Dazu zählen Füllstoffe, Formtrennmittel, Rieselhilfsmittel, Stabilisatoren sowie wasserlösliche oder wasserunlösliche Farbstoffe, Aromen und Süßstoffe.

Farbstoffe sind z.B. Eisenoxide, Titandioxid, die in einer Menge von etwa 0.001 bis 10, vorzugsweise von 0.5 bis 3 Gew.-% zugesetzt werden, Triphenylmethanfarbstoffe, Azofarbstoffe, Chinolinfarbstoffe, Indigofarbstoffe, Carotinoide, um die Kapseln einzufärben, Opakisierungsmittel wie Titandiodid oder Talkum, um die Lichtundurchlässigkeit zu erhöhen und um Farbstoffe einzusparen.

Aromen und Süßstoffe sind insbesondere dann von Bedeutung, wenn ein schlechter Geruch oder Geschmack überdeckt werden soll und die Kapsel zerbissen wird.

Konservierungsmittel sind in aller Regel nicht erforderlich.

Füllstoffe sind z.B. anorganische Füllstoffe wie Oxide von Magnesium, Aluminium, Silicium, Titan oder Calciumcarbonat. Der bevorzugte Konzentrationsbereich für die Füllstoffe ist etwa 1 bis 50 Gew.-%, besonders bevorzugt 2 bis 30 Gew.-% bezogen auf das Gesamtgewicht sämtlicher Komponenten.

Schmiermittel sind Stearate von Aluminium, Calcium, Magnesium und Zinn, sowie Magnesiumsilikat, Silikone und ähnliche. Der bevorzugte Konzentrationsbereich ist etwa 0.1 bis 5 Gew.-%, besonders bevorzugt etwa 0.1 bis 3 Gew.-% bezogen auf das Gesamtgewicht sämtlicher Komponenten.

Rieselhilfsmittel sind z.B. feinteilige bzw. feinstteilige Kieselsäuren, ggf. modifiziert. Der bevorzugte Konzentrationsbereich ist 0.05 bis 3 Gew.-%, besonders bevorzugt 0.1 bis 1 Gew.-% bezogen auf das Gesamtgewicht sämtlicher Komponenten.

Ein Sonderfall stellt die Einarbeitung von Wirkstoffen in die Hülle dar. Dies kann vorteilhaft sein, um inkompatible Wirkstoffe voneinander zu trennen. Der Wirkstoff mit der geringsten Dosierung sollte dann in die Hülle eingearbeitet werden.

Die erfindungsgemäß erhaltenen Weichkapselhüllen bestehen aus 10 bis 100 %, vorzugsweise 20 bis 98 % Polymerisaten, gegebenenfalls 0 bis 80 %, vorzugsweise 1 bis 50 % strukturverbessernden Hilfsstoffen und gegebenenfalls 0 bis 30 %, vorzugsweise 0,1 bis 30 % weiteren üblichen Bestandteilen.

Die Herstellung der befüllten Weichkapseln kann nach an sich für die Herstellung von Weichkapseln bekannten Verfahren erfolgen, beispielsweise nach dem Rotary-Die-Verfahren, dem Accogel-Verfahren, dem Norton-Verfahren, dem Tropf- oder Blasverfahren oder dem Colton-Upjohn-Verfahren. Diese Verfahren sind beschreiben in "W. Fahrig und U.Hofer, Die Kapsel, Wissenschaftliche Verlagsgesellschaft mbH, Suttgart, 1983".

Vor der Verarbeitung in der Verkapselungseinheit können die erfindungsgemäß erhaltenen als Weichkapselhüllen geeigneten Filme gewünschtenfalls mit Wasser oder mit Wasser mischbaren organischen Lösungsmitteln oder Gemischen aus Wasser und mit Wasser mischbaren Lösungsmitteln befeuchtet werden. Geeignete mit Wasser mischbare Lösungsmittel sind: Glycerin, 1,2-Propylenglykol, Polyethylenglykol mit Molekulargewichten zwischen 250 und 600. Dies empfiehlt sich insbesondere dann, wenn der zur Verkapselung verwendete Film nicht ausreichend weich und klebrig ist und somit die Verschweißung erschwert ist. Das oberflächliche Aufbringen dieser Stoffe erweicht den Film und verbessert die Verschweißbarkeit. Die Aufbringung kann durch Aufsprühen, Aufwalzen, Aufpinseln oder Aufrakeln erfolgen.

Die besonderen Vorzüge der beschriebenen Kapseln und des beschriebenen Verfahrens liegen darin, dass die Prozesszeiten für die Herstellung der Filme sehr kurz sind (nur wenige Minuten) und die Filme kaum Luftblasen enthalten. Durch die kurze Herstellungszeit kann die Geschwindigkeit der Filmherstellung an die Verkapselungsgeschwindigkeit adaptiert werden. Dadurch erfolgen Filmherstellung und Verkapselung in einem völlig kontinuierlichen Prozess. Dies ist unter Verwendung des Verfahrens von Filmziehung aus Polymerlösungen nicht möglich. Hier muss zunächst die Polymerlösung hergestellt werden, gerakelt und getrocknet werden.

Ferner ermöglicht das erfindungsgemäße Verfahren die einfache, individuelle Einstellung des Wassergehaltes nahezu ohne jegliche Einschränkung durch hohe Viskositäten. Dadurch sind sehr hohe Feststoffgehalte einstellbar. Demgegenüber kann das Rakeln von Filmen kann nur mit niedrigviskosen Lösungen erfolgen. Aufgrund der geringeren Menge an Wasser, die verdampft werden muss ergibt sich bei dem erfindungsgemäßen Verfahren eine wesentlich günstigere Energiebilanz.

Die Verschweißung kann ebenfalls mit hoher Geschwindigkeit sehr gleichmäßig und reproduzierbar und ohne Risse oder Poren erfolgen, wodurch der Ausschuss an beschädigten Kapseln extrem gering ist. Ferner sind die Kapeln leicht zu trocknen, behalten hierbei ihre Form und Flexibilität und sind lagerstabil, da sie nicht wie z.B. Stärke das Phänomen der Retrogradation aufweisen. Die Auflösegeschwindigkeit der Hülle ist größer als bei den bekannten Kapseln und vor allem lösen sie sich auch in kalten wässrigen Medien.

Typische verpackte Materialien sind bevorzugt pharmazeutische Erzeugnisse, wie feste und flüssige Wirkstoffe, aber auch Vitamine, Carotinoide, Mineralstoffe, Spurenelemente, Nahrungsergänzungsstoffe, Gewürze sowie Süßstoffe. Weiterhin können die Kapseln für kosmetische Wirkstoffe ("personal care"), wie beispielsweise Haar- und Hautformulierungen, für Öle, Duftstoffe, Badezusätze oder Proteine verwendet werden. Weitere Anwendungen im Bereich "personal care" sowie weitere Anwendungen für wasserlösliche Verpackungen sind in der WO 99/40156 genannt.

Weitere verpackte Materialien können sein, z.B. Reinigungsmittel, wie Seifen, Waschmittel, Farb- und Bleichmittel, Agrarchemikalien wie Düngemittel (-kombinationen), Pflanzenschutzmittel wie Herbizide, Fungizide oder Pestizide und Saatgut.

Generell lassen sich Inhaltsstoffe verpacken, die geschützt werden sollen, bevor sie in eine wässrige Umgebung gebracht werden.

Die erfindungsgemäß erhaltenen Weichkapseln der erfindungsgemäßen Zusammensetzung lassen sich hervorragend unter Verwendung von wässrigen Polymerlösungen oder Polymersuspensionen coaten. So kann durch Aufsprühen von Kollicoat MAE 30 DP (Methacrylsäure-Copolymer Typ C der USP) in einem Horizontaltrommelcoater ein stark auf der Oberfläche haftender magensaftresistenter Überzug aufgebracht werden, der zudem lagerungsstabil ist.

### Beispiele

Folgende Pfropf-Polymere können nach dem erfindungsgemäßen Verfahren verarbeitet werden:

### Allgemeine Herstellvorschrift:

In einem Polymerisationsgefäß wird die polyetherhaltige Verbindung vorgelegt und unter Rühren und leichtem Stickstoffstrom auf 80°C erhitzt. Unter Rühren werden Vinylacetat und das weitere Monomere in 3 h zudosiert. Gleichzeitig wird eine Lösung von 1,4 g tert.-Butylperpivalat in 30 g Methanol ebenfalls in 3 h zugegeben. Danach wird noch 2 h bei 80°C gerührt. Nach dem Abkühlen wird das Polymerisat in 450 ml Methanol gelöst. Zur Verseifung gibt man bei 30°C, 50 ml einer 10%igen methanolischen Natriumhydroxidlösung zu. Nach 40 min. wird die Reaktion durch Zugabe von 750 ml 1 %iger Essigsäure abgebrochen. Das Methanol wird durch Destillation entfernt.

Die K-Werte wurden 1 %ig in N-Methylpyrrolidon bestimmt.

| Polymer | Pfropfgrundlage | Vinylester | K-Wert | Verseifungsgrad [%] |
|---|---|---|---|---|
| 1 | PEG 1500¹ 72g | Vinylacetat, 410 g | 47 | > 96 |
| 2 | PEG 4000 72 g | Vinylacetat, 410 g | 51 | > 96 |
| 3 | PEG 6000, 72 g | Vinylacetat, 410 g | 54 | 95 |
| 4 | PEG 6000, 137 g | Vinylacetat, 410 g | 49 | 96 |
| 5 | PEG 6000, 22 g | Vinylacetat 410 g | 73 | 94 |
| 6 | PEG 6000, 410g | Vinylacetat 410 g | 42 | 96 |
| 7 | PEG 9000, 137 g | Vinylacetat, 410 g | 58 | 97 |
| 8 | Polyglycerin 2200, 72 g | Vinylacetat, 410 g | 66 | 96 |
| 9 | PEG-PPG-Blockcopolymer 8000², 72 g | Vinylacetat, 410 g | 45 | 96 |
| 10 | Methylpolyethylenglykol 2000³ 72 g | Vinylacetat, 410 g | 47 | 97 |
| 11 | Alkylpolyethylenglykol 3500⁴ 72 g | Vinylacetat, 410 g | 48 | 98 |
| 12 | PPG 4000⁵ 72 | Vinylacetat 410 g | 50 | 92 |
| 13 | PEG 20000 72 g | Vinylacetat, 410g | 69 | 96 |
| 14 | PEG 20000 103 g | Vinylacetat, 410 g | 64 | 94 |
| 15 | PEG 20000 137 g | Vinylacetat, 410 g | 59 | 95 |
| 16 | PEG 20000 615 g | Vinylacetat, 410 g | 55 | 86 |
| 17 | PEG 35000 72 g | Vinylacetat, 410 g | 77 | 95 |
| 18 | PEG 35000 137g | Vinylacetat, 410 g | 80 | 95 |
| 19 | PEG 35000 205 g | Vinylacetat, 410 g | 65 | 97 |
| 28 | PEG 35000, 270 g | Vinylacetat, 410 g | 59 | 96 |

| | | | | |
|---|---|---|---|---|
| 1 PEG x: Polyethylenglykol mit mittlerem Molekulargewicht x 2 Lutrol F 68 der Fa. BASF Aktiengesellschaft (PPG: Polypropylenglykol) 3 Pluriol A 2000 E der Fa. BASF Aktiengesellschaft 4 Lutensol AT 80 der Fa. BASF Aktiengesellschaft (C₁₆-C₁₈-Fettalkohol + 80 EO) 5 Polypropylenglykol mit mittlerem Molekulargewicht 4000 | | | | |

### Allgemeine Vorschrift zur Herstellung von Weichkapselhüllen:

Die Verarbeitung erfolgte in einem Zweischneckenkneter ZSK 25 der Firma Coperion Werner & Pfleiderer mit 8 Zylindern, der einen Schneckendurchmesser von 25 mm bei einem L/D-Verhältnis von 34:1 aufwies. Die Zylindertemperatur betrug 90 bis 120°C, die Schneckendrehzahl 120 bis 150 Upm.

Die Polymeren wurden in Zylinder 2 in den Extruder eingezogen und leicht erwärmt. Über Injektordüsen wurden die entsprechenden Mengen Wasser und Weichmacher in Zylinder 4 zudosiert und die Mischung zur Auflösung der Polymeren auf 97°C erhitzt. Die Extrusion erfolgte bei einer Düsentemperatur von 93°C durch eine Schlitzdüse mit einer Breite von 100 mm und einer Höhe von 600 µm. Die austretenden Filme wurden auf 47° C abgekühlt.

Die Prozesszeit für die Herstellung der Filme betrug 4min (Lösen des Polymers und Extrusion der Filme: 2 min, Abkühlung der Filme 2 min).
Die Filme enthielten keine Luftblasen.

Die Zusammensetzung der einzelnen Filme ist in der nachstehenden Tabelle aufgelistet.

Als Polymer A wurde das Pfropfpolymer 3 verwendet.
Als Polymer B wurde eine Mischung aus Pfropfpolymer 3 und Polyvinylalkohol (M_{w} 37.000) im Gewichtsverhältnis 6:4 verwendet.

| Beispiel Nr. | Zusammensetzung Extrusionslösung | | |
|---|---|---|---|
| | Polymer/ [Gew.-%] | Wasser [Gew.-%] | Hilfsstoff /[Gew.-%] |
| 1 | A/45 | 55 | 0 |
| 2 | A/50 | 50 | 0 |
| 3 | A/45 | 47,5 | Glycerin / 7,5 |
| 4 | B/50 | 45 | Glycerin / 5 |
| 5 | B / 56 | 38 | Glycerin / 6 |
| 6 | B/50 | 35 | Glycerin / 15 |
| 7 | B/58 | 37 | Glycerin / 5 |
| 8 | A/50 | 45 | Carrageenan / 5 |
| 9 | B / 50 | 45 | Hydroxypropylstärke/5 |
| 10 | A/45 | 55 | HPC/5 |

### Herstellung von Weichkapseln

Die Filme gemäß den Beispielen 1-10 wurden nach dem Rotary-die-Verfahren zu Weichkapseln der Größe 10 minims oval verarbeitet. Als Kapselfüllung wurde Tocopherolacetat verwendet, in einer Dosierung von 500mg. Die Kapseln wurden in einer Trommel gesammelt und 24 h bei 35°C getrocknet.

Der Ausschuss bei der Verkapselung an nicht sauber verschweißten Kapseln lag bei kleiner 1%.

### Vergleichsbeispiel

1,0 kg Polymer aus Polyethylenglykol 6000/Polyvinylacetat (15 : 85) verseift, hergestellt nach EP 1136070 wurden in 1,5 kg demineralisiertem Wasser gelöst und die auf 60°C erwärmte Lösung zu einem 300 µm dicken Film ausgezogen und bei 60°C getrocknet. Aus diesem Film wurden mittels des rotary-die-Verfahrens Weichgelatinekapseln mit ebenfalls 500 mg Tocopherolacetat hergestellt.

Die Prozesszeit für die Herstellung des Filmes betrug 125 min (Lösen des Polymers: 45 min, weitgehende Entfernung der Luftblasen: 60 min, Filmziehung und Trocknung: 20 min).

Die Kapselhülle enthielt noch sichtbare Luftblasen.

Der Ausschuss an nicht sauber verschweißten Kapseln betrug 11 %.

## Patentansprüche

1. Verfahren zur Herstellung von gelatinefreien Weichkapselhüllen basierend auf Polyvinylalkohol-Polyether-Pfropfpolymeren als Hüllpolymeren, **dadurch gekennzeichnet, dass** man eine wässrige Lösung, enthaltend mindestens 45 Gew.-%, bezogen auf das Gesamtgewicht der Lösung, einer Polymerkomponente, wobei die Polymerkomponente aus einem Polyvinylalkohol-Polyether-Pfropfpolymeren oder einer Mischung aus Polyvinylalkohol-Polyether-Pfropfpolymeren und Polyvinylalkohol besteht, erhitzt, die Lösung bei einer Temperatur von mindestens 70°C extrudiert und den entstandenen Film durch Abkühlung verfestigt.

2. Verfahren nach Anspruch 1, **dadurch gekennzeichnet, dass** der wässrigen Lösung zusätzlich weitere Hilfsstoffe zugesetzt werden.

3. Verfahren nach Anspruch 1 oder 2, **dadurch gekennzeichnet, dass** das Gewichtsverhältnis von Polyvinylalkohol-Polyether-Pfropfpolymeren zu Polyvinylalkohol 100:1 bis 30:70 beträgt.

4. Verfahren nach einem der Ansprüche 1 bis 3, **dadurch gekennzeichnet, dass** als weitere Hilfsstoffe Stärkederivate, Cellulosederivate oder Carrageenane eingesetzt werden.

5. Verfahren nach einem der Ansprüche 1 bis 4, **dadurch gekennzeichnet, dass** als weitere Hilfsstoffe Weichmacher eingesetzt werden.

6. Verfahren nach einem der Ansprüche 1 bis 5, **dadurch gekennzeichnet, dass** die Herstellung der wässrigen Lösung in einem Extruder erfolgt.

7. Verfahren nach einem der Ansprüche 1 bis 6, **dadurch gekennzeichnet, dass** die wässrige Lösung der Polymerkomponenten durch eine Schlitzdüse extrudiert wurde.

8. Verfahren nach einem der Ansprüche 1 bis 7, **dadurch gekennzeichnet, dass** die nach Abkühlung erhaltenen Filme vor Einbringen in die Verkapselungseinheit mit Wasser, einem mit Wasser mischbaren organischen Lösungsmittel oder einem Gemisch aus Wasser und einem mit Wasser mischbaren organischen Lösungsmittel angefeuchtet werden.

9. Verfahren nach einem der Ansprüche 1 bis 8, **dadurch gekennzeichnet, dass** die Extrudate zur Verfestigung auf eine Temperatur abgekühlt werden, die mindestens 10°C unter der Extrusionstemperatur liegen.

## Claims

1. A process for producing gelatin-free soft capsule shells based on polyvinyl alcohol-polyether graft copolymers as shell polymers, which comprises heating an aqueous solution comprising at least 45% by weight, based on the total weight of the solution, of a polymer component, where the polymer component consists of a polyvinyl alcohol-polyether graft copolymer or a mixture of polyvinyl alcohol-polyether graft copolymers and polyvinyl alcohol, extruding the solution at a temperature of at least 70°C, and solidifying the resulting film by cooling.

2. The process according to claim 1, wherein further auxiliaries are additionally added to the aqueous solution.

3. The process according to claim 1 or 2, wherein the ratio of polyvinyl alcohol-polyether graft copolymer to polyvinyl alcohol is from 100:1 to 30:70 by weight.

4. The process according to any of claims 1 to 3, wherein starch derivatives, cellulose derivatives or carrageenans are employed as further auxiliaries.

5. The process according to any of claims 1 to 4, wherein plasticizers are employed as further auxiliaries.

6. The process according to any of claims 1 to 5, wherein the aqueous solution is produced in an extruder.

7. The process according to any of claims 1 to 6, wherein the aqueous solution of the polymer component has been extruded through a slot die.

8. The process according to any of claims 1 to 7, wherein the films obtained after cooling are moistened with water, with a water-miscible organic solvent or with a mixture of water and a water-miscible organic solvent before introduction into the encapsulation unit.

9. The process according to any of claims 1 to 8, wherein the extrudates are solidified by cooling to a temperature which are at least 10°C below the extrusion temperature.

## Revendications

1. Procédé pour la fabrication d'enveloppes de gélules sans gélatine, à base de polymères greffés poly(alcool vinylique)-polyéther en tant que polymères d'enveloppe, **caractérisé en ce qu'**on chauffe une solution aqueuse, contenant au moins 45 % en poids, par rapport au poids total de la solution, d'un composant polymère, le composant polymère consistant en un polymère greffé poly(alcool vinylique)-polyéther ou un mélange de polymère greffé poly(alcool vinylique)-polyéther et poly(alcool vinylique), on extrude la solution à une température d'au moins 70 °C et on solidifie par refroidissement le film résultant.

2. Procédé selon la revendication 1, **caractérisé en ce qu'**en outre on ajoute d'autres adjuvants à la solution aqueuse.

3. Procédé selon la revendication 1 ou 2, **caractérisé en ce que** le rapport pondéral du polymère greffé poly(alcool vinylique)-polyéther au poly(alcool vinylique) vaut de 100:1 à 30:70.

4. Procédé selon l'une quelconque des revendications 1 à 3, **caractérisé en ce qu'**on ajoute comme autres adjuvants des dérivés d'amidon, des dérivés de cellulose ou des carraghénanes.

5. Procédé selon l'une quelconque des revendications 1 à 4, **caractérisé en ce qu'**on utilise comme autres adjuvants des plastifiants.

6. Procédé selon l'une quelconque des revendications 1 à 5, **caractérisé en ce que** la préparation de la solution aqueuse s'effectue dans une extrudeuse.

7. Procédé selon l'une quelconque des revendications 1 à 6, **caractérisé en ce que** la solution aqueuse des composants polymères a été extrudée à travers une filière plate.

8. Procédé selon l'une quelconque des revendications 1 à 7, **caractérisé en ce qu'**avant l'introduction dans l'unité d'encapsulation on humecte avec de l'eau, un solvant organique miscible à l'eau ou un mélange d'eau et d'un solvant organique miscible à l'eau les films obtenus après refroidissement.

9. Procédé selon l'une quelconque des revendications 1 à 8, **caractérisé en ce que** pour la solidification on refroidit les extrudats jusqu'à une température qui se situe à au moins 10 °C au-dessous de la température d'extrusion.
